(19) Europäisches Patentamt
European Patent Office
Office européen des brevets

(11) EP 4 289 936 A1

(12) # EUROPEAN PATENT APPLICATION
published in accordance with Art. 153(4) EPC

(43) Date of publication:
**13.12.2023 Bulletin 2023/50**

(21) Application number: **22749742.7**

(22) Date of filing: **02.02.2022**

(51) International Patent Classification (IPC):
***C12N 5/02*** *(2006.01)* ***C07K 5/00*** *(2006.01)*
***C07K 7/06*** *(2006.01)* ***C12M 3/00*** *(2006.01)*
***C12N 5/071*** *(2010.01)*

(52) Cooperative Patent Classification (CPC):
**C07K 5/00; C07K 7/06; C12M 1/00; C12M 3/00; C12N 5/0006; C12N 5/06**

(86) International application number:
**PCT/JP2022/004062**

(87) International publication number:
**WO 2022/168871 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.02.2021 JP 2021015724**
**31.08.2021 JP 2021141461**

(71) Applicant: **SEKISUI CHEMICAL CO., LTD.**
**Osaka-shi**
**Osaka**
**530-8565 (JP)**

(72) Inventors:
- **TAKAKURA, Kenta**
**Mishima-gun, Osaka 618-0021 (JP)**
- **HANEDA, Satoshi**
**Mishima-gun, Osaka 618-0021 (JP)**
- **NAKAMURA, Yuuta**
**Mishima-gun, Osaka 618-0021 (JP)**

(74) Representative: **Ter Meer Steinmeister & Partner Patentanwälte mbB**
**Nymphenburger Straße 4**
**80335 München (DE)**

Remarks:
The complete document including Reference Table(s) and the Sequence Listing(s) can be downloaded from the EPO website

(54) **CELL CULTURE MICROCARRIER AND CELL CULTURING METHOD**

(57) Provided is a cell culture microcarrier capable of suppressing the aggregation of a microcarrier due to cell clumps and enhancing the efficiency of culturing cells. The cell culture microcarrier according to the present invention includes a base particle and a coating layer coating the outer surface of the base particle, and has a specific gravity of 1.10 g/cm$^3$ or more.

[FIG. 1.]

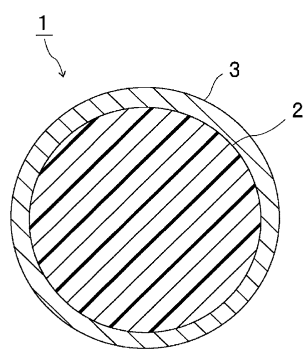

## Description

### TECHNICAL FIELD

The present invention relates to a cell culture microcarrier. The present invention also relates to a cell culturing method using the cell culture microcarrier.

### BACKGROUND ART

In research and development in academic fields, drug discovery fields, and regenerative medicine fields and the like, cells of animals such as human, mouse, rat, pig, cow, and monkey are used. As a method for culturing the cells, a method using a microcarrier is known.

Conventionally, as the microcarrier, a microcarrier in which a base particle is coated with an extracellular matrix (ECM) is widely used. For example, Patent Document 1 below describes a microcarrier including a polystyrene particle and vitronectin disposed on the outer surface of the polystyrene particle.

A microcarrier in which a base particle is coated with a synthetic resin is also known. For example, Patent Document 2 below describes a microcarrier including a polystyrene particle and a synthetic resin layer disposed on the outer surface of the polystyrene particle. The synthetic resin layer contains a synthetic resin to which a peptide is bonded.

### Related Art Document

### Patent Document

Patent Document 1: WO2011/017167A1
Patent Document 2: WO2011/017050A1

### SUMMARY OF THE INVENTION

### PROBLEMS TO BE SOLVED BY THE INVENTION

In the conventional microcarriers as described in Patent Documents 1 and 2, the particle having a small specific gravity such as the polystyrene particle is used as the base particle, and thus the specific gravity of the microcarrier itself is also small. By using the microcarrier having a small specific gravity, cells can be cultured while the microcarrier is satisfactorily suspended in a culture medium.

Incidentally, in a cell culturing method using a microcarrier, the microcarrier to which the cells adhere may be settled by gravity, and a supernatant culture medium may be then removed, followed by adding a new culture medium for culture medium replacement.

In the conventional microcarrier having a small specific gravity, a difference is apt to occur in the settling time of the microcarrier when the microcarrier is settled by gravity. For example, the conventional microcarrier having a small specific gravity includes a microcarrier that is relatively quickly settled to the bottom of a culture vessel, and a microcarrier that takes a relatively long time to be settled to the bottom of the culture vessel. In this case, before substantially the entire amounts of the microcarriers are settled to the bottom of the culture vessel, cells adhering to the microcarriers that have been previously settled may form cell clumps between the microcarriers, which causes the microcarriers to aggregate. As a result, the efficiency of culturing the cells is reduced.

An object of the present invention is to provide a cell culture microcarrier capable of suppressing the aggregation of a microcarrier due to cell clumps and enhancing the efficiency of culturing cells. Another object of the present invention is to provide a cell culturing method using the cell culture microcarrier.

### MEANS FOR SOLVING THE PROBLEMS

According to a broad aspect of the present invention, there is provided a cell culture microcarrier (hereinafter, may be abbreviated as a microcarrier) including: a base particle; and a coating layer coating an outer surface of the base particle, the cell culture microcarrier having a specific gravity of 1.10 g/cm$^3$ or more.

In a specific aspect of the microcarrier according to the present invention, the microcarrier has a water absorption of 10% by weight or less.

In a specific aspect of the microcarrier according to the present invention, the coating layer contains a synthetic

resin.

In a specific aspect of the microcarrier according to the present invention, the synthetic resin has a polyvinyl alcohol derivative skeleton or a poly(meth)acrylic acid ester skeleton.

In a specific aspect of the microcarrier according to the present invention, the coating layer contains a peptide moiety.

In a specific aspect of the microcarrier according to the present invention, the base particle is a resin particle.

In a specific aspect of the microcarrier according to the present invention, the base particle contains a polymer of a monomer having an ethylenically unsaturated group.

In a specific aspect of the microcarrier according to the present invention, the polymer of a monomer having an ethylenically unsaturated group is an acrylic resin, a divinylbenzene polymer, or a divinylbenzene copolymer.

In a specific aspect of the microcarrier according to the present invention, the microcarrier has an average particle diameter of 100 um or more and 1500 um or less.

In a specific aspect of the microcarrier according to the present invention, a particle diameter CV value is 10% or less.

According to a broad aspect of the present invention, there is provided a cell culturing method including the steps of: causing a cell to adhere to the cell culture microcarrier described above; settling the cell culture microcarrier to which the cell adheres; and replacing a culture medium.

## EFFECT OF THE INVENTION

A cell culture microcarrier according to the present invention includes a base particle and a coating layer coating an outer surface of the base particle. The cell culture microcarrier according to the present invention has a specific gravity of 1.10 g/$cm^3$ or more. The cell culture microcarrier according to the present invention has the above configuration, which makes it possible to suppress the aggregation of the microcarrier due to cell clumps and to enhance the efficiency of culturing cells.

## BRIEF DESCRIPTION OF DRAWINGS

[Fig. 1] Fig. 1 is a cross-sectional view schematically showing a cell culture microcarrier according to an embodiment of the present invention.

## MODE FOR CARRYING OUT THE INVENTION

Hereinafter, the present invention will be described in detail.

(Cell culture microcarrier)

A cell culture microcarrier (hereinafter, may be abbreviated as a "microcarrier") according to the present invention includes a base particle and a coating layer coating an outer surface of the base particle. The microcarrier according to the present invention has a specific gravity of 1.10 g/$cm^3$ or more.

The microcarrier according to the present invention has the above configuration, which makes it possible to suppress the aggregation of the microcarrier due to cell clumps and to enhance the efficiency of culturing cells.

Conventionally, a microcarrier having a small specific gravity (for example, a microcarrier having a specific gravity of about 1.02 g/$cm^3$) have been used from the viewpoint of culturing cells while suspending the microcarrier in a culture medium. However, in the conventional microcarrier having a small specific gravity, a difference is apt to occur in the settling time of the microcarrier in culture medium replacement. For this reason, in the conventional microcarrier having a small specific gravity, cells adhering to the microcarriers that have been previously settled may form cell clumps between the microcarriers, which causes the microcarriers to aggregate. This results in a decrease in the efficiency of culturing the cells.

In contrast, the microcarrier according to the present invention has a moderately large specific gravity. The microcarrier according to the present invention includes the base particle and the coating layer. In the microcarrier according to the present invention, by adopting the above-described configuration, the aggregation of the microcarrier due to the cell clumps can be suppressed. For example, the microcarrier according to the present invention can be settled to the bottom of a culture vessel in a short time by gravity in culture medium replacement. Therefore, a time required for culture medium replacement can be shortened, and the aggregation of the microcarrier due to the cell clumps can be suppressed. Therefore, in the microcarrier according to the present invention, the efficiency of culturing the cells can be enhanced.

In the microcarrier according to the present invention, it is not necessary to use a natural polymer material such

as an extracellular matrix (ECM) as a material, so that the microcarrier is inexpensive, has little variation between lots, and is excellent in safety.

From the viewpoint of exerting the effect of the present invention, the specific gravity of the microcarrier is 1.10 g/cm$^3$ or more.

The specific gravity of the microcarrier is preferably 1.12 g/cm$^3$ or more, and more preferably 1.15 g/cm$^3$ or more, and preferably 6.00 g/cm$^3$ or less, more preferably 5.00 g/cm$^3$ or less, still more preferably 4.00 g/cm$^3$ or less, and particularly preferably 2.00 g/cm$^3$ or less. The specific gravity of the microcarrier is preferably 1.12 g/cm$^3$ or more and 6.00 g/cm$^3$ or less, more preferably 1.15 g/cm$^3$ or more and 5.00 g/cm$^3$ or less, still more preferably 1.15 g/cm$^3$ or more and 4.00 g/cm$^3$ or less, and particularly preferably 1.15 g/cm$^3$ or more and 2.00 g/cm$^3$ or less. When the specific gravity is the lower limit or more, the settling time of the microcarrier in culture medium replacement can be further shortened, and as a result, the aggregation of the microcarrier due to the cell clumps can be more effectively suppressed. When the specific gravity is the above upper limit or less, the cells can be cultured while the microcarrier to which the cells adhere are satisfactorily suspended in the culture medium by appropriate shaking or the like. When the specific gravity is the upper limit or less, a swirling property provided by a stirring blade can be improved.

The specific gravity of the microcarrier is measured using a true specific gravity meter.

The water absorption of the microcarrier is preferably 10% by weight or less, more preferably 5% by weight or less, and still more preferably 1% by weight or less. When the water absorption is the above upper limit or less, the state of the surface of the microcarrier is less likely to change at the time of the adhesion of the cells, and thus variation in an initial fixing ratio after cell seeding can be reduced. When the water absorption is the above upper limit or less, the cells are less likely to be released from the microcarrier in the culture medium. The lower limit of the water absorption of the microcarrier is not particularly limited. The water absorption of the microcarrier may be 0% by weight or more, or 0.001% by weight or more.

The water absorption of the microcarrier can be measured as follows.

A microcarrier dried in an oven at 100°C for 8 hours is prepared. One hundred point zero mg of the microcarrier is allowed to stand for 24 hours in an environment of a temperature of 37°C and a relative humidity of 95% RH. The weight of the microcarrier after standing is measured. The water absorption of the microcarrier is calculated by the following formula.

$$\text{Water absorption (\% by weight)} = (W_2 - W_1)/W_1 \times 100$$

$W_1$: Weight of microcarrier before standing (mg)
$W_2$: Weight of microcarrier after standing (mg)

Examples of a method for reducing the water absorption of the microcarrier include producing a coating layer using a material having high hydrophobicity.

The average particle diameter of the microcarrier is preferably 100 um or more, more preferably 150 um or more, still more preferably 200 um or more, yet still more preferably 250 um or more, and particularly preferably 300 um or more, and preferably 1500 um or less, more preferably 1000 um or less, still more preferably 800 um or less, yet still more preferably 700 um or less, and particularly preferably 500 um or less. The average particle diameter of the microcarrier is preferably 100 um or more and 1500 um or less, more preferably 150 um or more and 1000 um or less, still more preferably 200 um or more and 800 um or less, yet still more preferably 250 um or more and 700 um or less, and particularly preferably 300 um or more and 500 um or less. When the average particle diameter is the above lower limit or more, the effect of the present invention can be more effectively exhibited. When the average particle diameter is the above upper limit or less, cell clumps can be formed with a more uniform thickness on the surface of each microcarrier. When the average particle diameter is the above upper limit or less, an area to which the cells can adhere can be further increased.

The particle diameter of the microcarrier means the diameter of the microcarrier when the microcarrier is a true sphere, and means the diameter of the microcarrier when the microcarrier is assumed to be a true sphere corresponding to the volume of the microcarrier having a shape other than the true sphere.

The average particle diameter of the microcarrier is preferably a number average particle diameter. The average particle diameter of the microcarrier is determined by observing optional 50 microcarriers with an electron microscope or an optical microscope and calculating the average value of the particle diameters of the microcarriers, or using a particle size distribution measuring apparatus. In the observation with the electron microscope or the optical microscope, the particle diameter of one microcarrier is determined as a particle diameter in terms of an equivalent circle diameter. In the observation with the electron microscope or the optical microscope, the average particle diameter of optional 50 microcarriers in terms of an equivalent circle diameter is substantially equal to an average particle diameter in terms of an equivalent sphere diameter. In the particle size distribution measuring apparatus, the particle diameter of one microcarrier is determined as a particle diameter in terms of an equivalent sphere diameter. The average particle diameter of the microcarrier is preferably calculated using the particle size distribution measuring apparatus.

The coefficient of variation (CV value) of the particle diameter of the microcarrier is preferably 10% or less, more preferably 8% or less, still more preferably 50 or less, and particularly preferably 3% or less. When the coefficient of variation (CV value) is the above upper limit or less, the uniformity of a sedimentation rate can be improved, and the effect of the present invention can be more effectively exhibited. The coefficient of variation (CV value) of the particle diameter of the microcarrier may be 0% or more, 0.1% or more, 0.5% or more, or 1% or more. The coefficient of variation (CV value) of the particle diameter of the microcarrier may be 0% or more and 10% or less, 0.1% or more and 8% or less, 0.1% or more and 50 or less, or 1% or more and 3% or less.

The coefficient of variation (CV value) of the particle diameter of the microcarrier is calculated as follows.

$$\text{CV value (\%)} = (\rho/Dn) \times 100$$

ρ: standard deviation of particle diameter of microcarrier
Dn: average particle diameter of microcarrier

Examples of a method for reducing the coefficient of variation (CV value) of the particle diameter of the microcarrier include a dry classification method and a wet classification method.

The shape of the microcarrier is not particularly limited. The shape of the microcarrier may be spherical shape, may be a shape other than spherical shape, or may be a flat shape or the like. The spherical shape is not limited to a perfect spherical shape, and includes a substantially spherical shape, for example, a shape having an aspect ratio (major axis/minor axis) of 1.5 or less.

Hereinafter, the present invention will be specifically described with reference to the drawings.

Fig. 1 is a cross-sectional view schematically showing a cell culture microcarrier according to an embodiment of the present invention.

A cell culture microcarrier 1 shown in Fig. 1 includes a base particle 2 and a coating layer 3 coating an outer surface of the base particle 2. The coating layer 3 is disposed on the surface of the base particle 2 and is in contact with the surface of the base particle 2. The coating layer 3 coats the entire outer surface of the base particle 2.

Other details of the microcarrier will be described below.

In the present specification, "(meth)acrylate" means one or both of "acrylate" and "methacrylate", and "(meth)acryl" means one or both of "acryl" and "methacryl".

(Base particle)

The material of the base particle is not particularly limited as long as the specific gravity of the microcarrier is 1.10 g/cm$^3$ or more. The material of the base particle may be an organic material, an inorganic material, or both an organic material and an inorganic material. The base particle may contain a resin, may contain an inorganic filler, may contain a resin and an inorganic filler, or may not contain a resin. The base particle may be a resin particle or an inorganic particle. The base particle preferably contains a resin. The base particle is preferably a resin particle because it is easily produced. As the material of the base particle, only one kind may be used, or two or more kinds thereof may be used in combination. As the resin, only one kind may be used, or two or more kinds thereof may be used in combination.

Examples of the resin include a polyolefin resin, an acrylic resin, polycarbonate, polyamide, a phenol formaldehyde resin, a melamine formaldehyde resin, a benzoguanamine formaldehyde resin, a urea formaldehyde resin, a phenol resin, a melamine resin, a benzoguanamine resin, a urea resin, an epoxy resin, an unsaturated polyester resin, a saturated polyester resin, polyethylene terephthalate, polysulfone, polyphenylene oxide, polyacetal, polyimide, polyamideimide, polyetheretherketone, polyethersulfone, a divinylbenzene polymer, and a divinylbenzene copolymer.

The resin is preferably a polymer of a monomer having an ethylenically unsaturated group. The base particle preferably contains a polymer of a monomer having an ethylenically unsaturated group. In this case, the specific gravity of the base particle can be satisfactorily adjusted, and as a result, the specific gravity of the microcarrier can be adjusted to a suitable range.

Examples of the polymer of a monomer having an ethylenically unsaturated group include an acrylic resin, a divinylbenzene polymer, and a divinylbenzene copolymer. As the monomer having an ethylenically unsaturated group, only one kind may be used, or two or more kinds thereof may be used in combination.

The polymer of a monomer having an ethylenically unsaturated group is preferably an acrylic resin, a divinylbenzene polymer, or a divinylbenzene copolymer. In this case, the specific gravity of the base particle can be satisfactorily adjusted, and as a result, the specific gravity of the microcarrier can be adjusted to a suitable range.

When the base particle contains the polymer of a monomer having an ethylenically unsaturated group, the

polymer of a monomer having an ethylenically unsaturated group preferably has a crosslinked structure. In this case, the specific gravity of the base particle can be satisfactorily adjusted, and as a result, the specific gravity of the microcarrier can be adjusted to a suitable range.

Examples of a method for forming the crosslinked structure include the following methods: (1) a method for polymerizing a polymerizable component containing a monomer having two or more ethylenically unsaturated groups; and (2) a method for forming a crosslinked structure by reacting a polymer of a monomer having an ethylenically unsaturated group with a crosslinking agent.

In the method (1), examples of the monomer having two or more ethylenically unsaturated groups include divinylbenzene, polyfunctional (meth)acrylate, triallyl (iso)cyanurate, triallyl trimellitate, diallyl phthalate, and diallyl acrylamide. As the monomer having two or more ethylenically unsaturated groups, only one kind may be used, or two or more kinds thereof may be used in combination.

In the method (1), the polymerizable component may contain other monomer having an ethylenically unsaturated group. Examples of the other monomer having an ethylenically unsaturated group include styrene, monofunctional (meth)acrylate, (meth)acrylic acid, acrylonitrile, and vinyl chloride. As the other monomer having an ethylenically unsaturated group, only one kind may be used, or two or more kinds thereof may be used in combination.

Examples of a polymer obtained by the method (1) include a copolymer of divinylbenzene and styrene and a copolymer of polyfunctional (meth)acrylate and monofunctional (meth)acrylate.

Examples of the method (2) include a method in which a polymerizable component containing a monomer having an ethylenically unsaturated group and a functional group containing active hydrogen in the molecule is polymerized to obtain a polymer, and the polymer(s) is/are then crosslinked using a crosslinking agent.

Examples of the functional group containing active hydrogen include a hydroxyl group, a carboxyl group, an amino group, and a phenol group. Examples of the monomer having an ethylenically unsaturated group and a functional group containing active hydrogen in the molecule include hydroxyl group-containing (meth)acrylate, (meth)acrylic acid, and amino group-containing (meth)acrylate. As the monomer having an ethylenically unsaturated group and a functional group containing active hydrogen in the molecule, only one kind may be used, or two or more kinds thereof may be used in combination.

The crosslinking agent is not particularly limited as long as it can react with the functional group containing active hydrogen, and examples thereof include a polyfunctional isocyanate compound and a polyfunctional epoxy compound. As the crosslinking agent, only one kind may be used, or two or more kinds thereof may be used in combination.

As long as the specific gravity of the obtained microcarrier is 1.10 g/cm$^3$ or more, the method for forming the crosslinked structure is not limited to these methods.

The base particle can be obtained, for example, by polymerizing the monomer having an ethylenically unsaturated group. The polymerization method is not particularly limited, and examples thereof include known methods such as radical polymerization, ion polymerization, polycondensation (condensation polymerization), addition condensation, living polymerization, and living radical polymerization. Other polymerization methods include suspension polymerization in the presence of a radical polymerization initiator.

The base particle may contain an inorganic filler. For example, the specific gravity of the base particle and the microcarrier can be suitably increased by using the inorganic filler and a resin having a small specific gravity in combination.

Examples of the inorganic filler include carbon black, a glass filler, and a metal filler. As the inorganic filler, only one kind may be used, or two or more kinds thereof may be used in combination.

The content of the resin in 100% by weight of the base particle is preferably 80% by weight or more, more preferably 90% by weight or more, still more preferably 95% by weight or more, yet still more preferably 97% by weight or more, further preferably 99% by weight or more, and most preferably 100% by weight (whole amount). The content of the resin in 100% by weight of the base particle may be 100% by weight or less or less than 100% by weight.

The content of the polymer of the monomer having an ethylenically unsaturated group in 100% by weight of the base particle is preferably 80% by weight or more, more preferably 90% by weight or more, still more preferably 95% by weight or more, yet still more preferably 97% by weight or more, further preferably 99% by weight or more, and most preferably 100% by weight (whole amount). The content of the polymer of the monomer having an ethylenically unsaturated group in 100% by weight of the base particle may be 100% by weight or less or less than 100% by weight.

When the base particle is the inorganic particle, examples of the inorganic particle include a glass particle and a metal particle.

The specific gravity of the base particle is preferably 1.10 g/cm$^3$ or more, more preferably 1.12 g/cm$^3$ or more, and still more preferably 1.15 g/cm$^3$ or more, and preferably 6.00 g/cm$^3$ or less, more preferably 5.00 g/cm$^3$ or less, still more preferably 4.00 g/cm$^3$ or less, and particularly preferably 2.00 g/cm$^3$ or less. When the specific gravity is the lower limit or more and the upper limit or less, the specific gravity of the microcarrier can be adjusted to a suitable range.

The specific gravity of the base particle is measured using a true specific gravity meter.

The average particle diameter of the base particle is preferably 100 um or more, more preferably 150 um or

more, still more preferably 200 um or more, yet still more preferably 250 um or more, further preferably 300 um or more, and particularly preferably 350 um or more, and preferably 1500 um or less, more preferably 1000 um or less, still more preferably 800 um or less, yet still more preferably 700 um or less, and particularly preferably 500 um or less. The average particle diameter of the base particle is preferably 100 um or more and 1500 um or less, more preferably 150 um or more and 1000 um or less, still more preferably 200 um or more and 800 um or less, yet still more preferably 250 um or more and 700 um or less, further preferably 300 um or more and 500 um or less, and particularly preferably 350 um or more and 500 um or less. When the average particle diameter is the above lower limit or more, the effect of the present invention can be more effectively exhibited. When the average particle diameter is the above upper limit or less, cell clumps can be formed with a more uniform thickness on the surface of each microcarrier. When the average particle diameter is the above upper limit or less, an area to which the cells can adhere can be further increased.

The particle diameter of the base particle means the diameter of the base particle when the base particle is a true sphere, and means the diameter of the base particle when the base particle is assumed to be a true sphere corresponding to the volume of the base particle having a shape other than the true sphere.

The average particle diameter of the base particle is preferably a number average particle diameter. The average particle diameter of the base particle is determined by observing optional 50 base particles with an electron microscope or an optical microscope and calculating the average value of the particle diameters of the base particles or using a particle size distribution measuring apparatus. In the observation with the electron microscope or the optical microscope, the particle diameter of one base particle is determined as a particle diameter in terms of an equivalent circle diameter. In the observation with the electron microscope or the optical microscope, the average particle diameter of optional 50 base particles in terms of an equivalent circle diameter is substantially equal to an average particle diameter in terms of an equivalent sphere diameter. In the particle size distribution measuring apparatus, the particle diameter of one base particle is determined as a particle diameter in terms of an equivalent sphere diameter. The average particle diameter of the base particle is preferably calculated using the particle size distribution measuring apparatus.

(Coating layer)

The microcarrier includes a base particle and a coating layer coating an outer surface of the base particle. The coating layer is a layer composed of a component different from the component of the base particle. Examples of the component constituting the coating layer include a peptide and a synthetic resin. The coating layer preferably contains a synthetic resin. As the synthetic resin, only one kind may be used, or two or more kinds thereof may be used in combination.

From the viewpoint of enhancing the adhesiveness between the microcarrier and the cell and maintaining the low swelling degree of the microcarrier, the synthetic resin preferably has a polyvinyl alcohol derivative skeleton or a poly(meth)acrylic acid ester skeleton. In this case, the synthetic resin may have a polyvinyl alcohol derivative skeleton, may have a poly(meth)acrylic acid ester skeleton, or may have a polyvinyl alcohol derivative skeleton and a poly(meth)acrylic acid ester skeleton.

From the viewpoint of enhancing the adhesiveness between the microcarrier and the cell, the coating layer preferably contains a peptide moiety, and more preferably contains a synthetic resin having a peptide moiety (peptide skeleton). That is, from the viewpoint of enhancing the adhesiveness between the microcarrier and the cell, the synthetic resin more preferably has a peptide moiety. The aspect in which the coating layer contains a peptide moiety includes not only an aspect in which the coating layer contains a synthetic resin having a peptide moiety but also an aspect in which the coating layer contains only a peptide. From the viewpoint of further enhancing the adhesiveness between the microcarrier and the cell, the synthetic resin preferably has a polyvinyl alcohol derivative skeleton or a poly(meth)acrylic acid ester skeleton, and a peptide moiety.

In the present specification, the "resin having a polyvinyl alcohol derivative skeleton or a poly(meth)acrylic acid ester skeleton and a peptide moiety" may be described as a "peptide-conjugated resin". The peptide-conjugated resin may have a polyvinyl alcohol derivative skeleton and a peptide moiety, may have a poly(meth)acrylic acid ester skeleton and a peptide moiety, or may have a polyvinyl alcohol derivative skeleton, a poly(meth)acrylic acid ester skeleton, and a peptide moiety.

In the peptide-conjugated resin having a polyvinyl alcohol derivative skeleton, the polyvinyl alcohol derivative skeleton and the peptide moiety are preferably bonded to each other via a linker moiety. Therefore, the peptide-conjugated resin having a polyvinyl alcohol derivative skeleton preferably has a polyvinyl alcohol derivative skeleton, a peptide moiety, and a linker moiety.

In the peptide-conjugated resin having a poly(meth)acrylic acid ester skeleton, the poly(meth)acrylic acid ester skeleton and the peptide moiety may be bonded to each other via the linker moiety, or may be directly bonded to each other without the linker moiety. The peptide-conjugated resin having a poly(meth)acrylic acid ester skeleton may have a poly(meth)acrylic acid ester skeleton, a peptide moiety, and a linker moiety.

<Polyvinyl alcohol derivative skeleton>

The polyvinyl alcohol derivative skeleton is a skeleton portion derived from a polyvinyl alcohol derivative. The polyvinyl alcohol derivative is a compound derived from polyvinyl alcohol. From the viewpoint of further enhancing the adhesiveness between the microcarrier and the cell, the polyvinyl alcohol derivative is preferably a polyvinyl acetal resin, and the polyvinyl alcohol derivative skeleton is preferably a polyvinyl acetal skeleton. That is, the synthetic resin preferably has the polyvinyl acetal skeleton and the peptide moiety. As each of the polyvinyl alcohol derivative and the polyvinyl acetal resin, only one kind may be used, and two or more kinds thereof may be used in combination.
The polyvinyl alcohol derivative skeleton and the polyvinyl acetal skeleton preferably have an acetal group, a hydroxyl group, and an acetyl group in side chains. However, the polyvinyl alcohol derivative skeleton and the polyvinyl acetal skeleton may not have, for example, an acetyl group. For example, when all of the acetyl groups of the polyvinyl alcohol derivative skeleton and the polyvinyl acetal skeleton are bonded to the linker, the polyvinyl alcohol derivative skeleton and the polyvinyl acetal skeleton may not have an acetyl group.
The polyvinyl acetal resin can be synthesized by acetalizing polyvinyl alcohol with an aldehyde.
The aldehyde used for acetalizing polyvinyl alcohol is not particularly limited. Examples of the aldehyde include an aldehyde having 1 to 10 carbon atoms. The aldehyde may or may not have a chain aliphatic group, a cyclic aliphatic group or an aromatic group. The aldehyde may be a chain aldehyde or a cyclic aldehyde. As the aldehyde, only one kind may be used, or two or more kinds thereof may be used in combination.
From the viewpoint of further enhancing the adhesiveness between the microcarrier and the cell, the aldehyde is preferably formaldehyde, acetaldehyde, propionaldehyde, butyraldehyde, or pentanal, and more preferably butyraldehyde. Therefore, the polyvinyl acetal resin is more preferably a polyvinyl butyral resin, the polyvinyl acetal skeleton is more preferably a polyvinyl butyral skeleton, and the synthetic resin more preferably has a polyvinyl butyral skeleton.
In the synthetic resin, the acetalization degree of the polyvinyl alcohol derivative skeleton and the polyvinyl acetal skeleton (the butyralization degree in the case of the polyvinyl butyral resin) is preferably 40 mol% or more, and more preferably 50 mol% or more, and preferably 90 mol% or less, and more preferably 85 mol% or less. When the acetalization degree is the above lower limit or more, the fixability of the cells can be further enhanced, and the cells efficiently proliferate. When the acetalization degree is the above upper limit or less, solubility in a solvent can be improved.
In the synthetic resin, the content rate of hydroxyl groups (the amount of hydroxyl groups) in the polyvinyl alcohol derivative skeleton and the polyvinyl acetal skeleton is preferably 15 mol% or more, and more preferably 20 mol% or more, and preferably 45 mol% or less, more preferably 30 mol% or less, and still more preferably 25 mol% or less.
In the synthetic resin, the acetylation degree (the amount of acetyl groups) in the polyvinyl alcohol derivative skeleton and the polyvinyl acetal skeleton is preferably 1 mol% or more, and more preferably 2 mol% or more, and preferably 5 mol% or less, and more preferably 4 mol% or less. When the acetylation degree is the above lower limit or more and the above upper limit or less, the reaction efficiency between the polyvinyl acetal resin and the linker can be enhanced.
The acetalization degree, the acetylation degree, and the amount of hydroxyl groups of the polyvinyl alcohol derivative skeleton and the polyvinyl acetal skeleton can be measured by $^1$H-NMR (nuclear magnetic resonance spectrum).

<Poly(meth)acrylic acid ester skeleton>

The poly(meth)acrylic acid ester skeleton is a skeleton portion derived from a poly(meth)acrylic acid ester. The poly(meth)acrylic acid ester is obtained by polymerizing a (meth)acrylic acid ester. The poly(meth)acrylic acid ester skeleton has a skeleton derived from a (meth)acrylic acid ester. As the poly (meth) acrylic acid ester, only one kind may be used, or two or more kinds thereof may be used in combination.
Examples of the (meth)acrylic acid ester include (meth)acrylic acid alkyl ester, (meth)acrylic acid cyclic alkyl ester, (meth)acrylic acid aryl ester, (meth)acrylic acid polyethylene glycols, and (meth)acrylic acid phosphorylcholine. As the (meth) acrylic acid ester, only one kind may be used, or two or more kinds thereof may be used in combination.
Examples of the (meth)acrylic acid alkyl ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-octyl (meth)acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, lauryl (meth)acrylate, stearyl (meth)acrylate, and isotetradecyl (meth)acrylate.
The (meth)acrylic acid alkyl ester may be substituted with a substituent such as an alkoxy group having 1 to 3 carbon atoms and a tetrahydrofurfuryl group. Examples of such (meth)acrylic acid alkyl ester include methoxyethyl acrylate and tetrahydrofurfuryl acrylate.
Examples of the (meth)acrylic acid cyclic alkyl ester include cyclohexyl (meth)acrylate and isobornyl (meth)acrylate.
Examples of the (meth)acrylic acid aryl ester include phenyl (meth)acrylate and benzyl (meth)acrylate.

Examples of the (meth)acrylic acid polyethylene glycols include methoxy-polyethylene glycol (meth)acrylate, ethoxypolyethylene glycol (meth)acrylate, hydroxy-polyethylene glycol (meth)acrylate, methoxy-diethylene glycol (meth)acrylate, ethoxy-diethylene glycol (meth)acrylate, hydroxy-diethylene glycol (meth)acrylate, methoxy-triethylene glycol (meth)acrylate, ethoxy-triethylene glycol (meth)acrylate, and hydroxytriethylene glycol (meth)acrylate.

Examples of the phosphorylcholine (meth)acrylate include 2-(meth)acryloyloxyethyl phosphorylcholine.

The synthetic resin preferably has a structural unit derived from a (meth)acrylate compound (A) represented by the following formula (A1) or the following formula (A2). The poly(meth)acrylic acid ester skeleton preferably has a structural unit derived from the (meth)acrylate compound (A) represented by the following formula (A1) or the following formula (A2). Thereby, the hydrophobicity of the coating layer can be increased, and therefore the water absorption of the microcarrier can be further reduced. Therefore, variation in an initial fixing ratio after cell seeding can be reduced, and the cells are less likely to be released from the microcarrier in the culture medium. The (meth)acrylate compound (A) may contain a (meth)acrylate compound represented by the following formula (A1), may contain a (meth)acrylate compound represented by the following formula (A2), or may contain both a (meth)acrylate compound represented by the following formula (A1) and a (meth)acrylate compound represented by the following formula (A2). When the (meth)acrylate compound (A) contains both the (meth)acrylate compound represented by the following formula (A1) and the (meth)acrylate compound represented by the following formula (A2), R in the following formula (A1) and R in the following formula (A2) may be the same or different. As (meth)acrylate compound (A), only one kind may be used, or two or more kinds thereof may be used in combination. As each of the (meth)acrylate compound represented by the following formula (A1) and the (meth)acrylate compound represented by the following formula (A2), only one kind may be used, or two or more kinds thereof may be used in combination.

[Chemical 1]

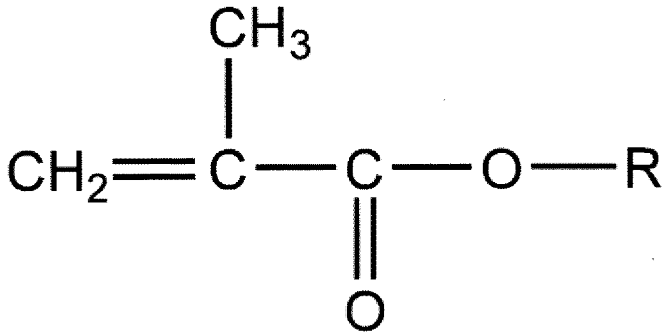

···(A1)

In the formula (A1), R represents a hydrocarbon group having 2 to 18 carbon atoms.

[Chemical 2]

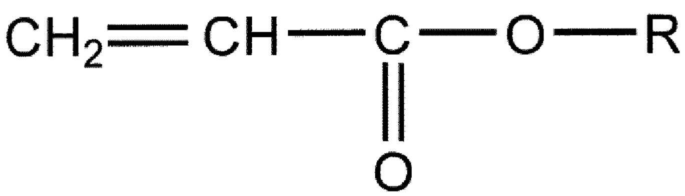

···(A2)

In the formula (A2), R represents a hydrocarbon group having 2 to 18 carbon atoms.

R in the formula (A1) and R in the formula (A2) may each be an aliphatic hydrocarbon group or an aromatic hydrocarbon group. From the viewpoint of improving the solubility of the synthetic resin, each of R in the formula (A1) and R in the formula (A2) is preferably an aliphatic hydrocarbon group. The aliphatic hydrocarbon group may be linear, may have a branched structure, may have a double bond, or may not have a double bond. R in the formula (A1) and R

in the formula (A2) may each be an alkyl group or an alkylene group.

The number of carbon atoms of R in the formula (A1) and the number of carbon atoms of R in the formula (A2) are each preferably 4 or more, more preferably 6 or more, still more preferably 8 or more, and particularly preferably 10 or more, and preferably 16 or less, more preferably 14 or less, and most preferably 12. When the number of carbon atoms is the above lower limit or more, the hydrophobicity of the synthetic resin can be further increased, and therefore the water absorption of the microcarrier can be further reduced. When the number of carbon atoms is the above upper limit or less, coatability when the material of the coating layer is disposed on the surface of the base particle can be enhanced. In particular, when the number of carbon atoms is 12, the water absorption of the microcarrier can be further reduced, and the coatability can be further enhanced.

The (meth)acrylic acid alkyl ester is preferably the (meth)acrylate compound (A).

The synthetic resin having a poly(meth)acrylic acid ester skeleton may have a skeleton derived from a monomer other than the (meth)acrylic acid ester.

Examples of the monomer other than the (meth)acrylic acid ester include (meth)acrylamides and vinyl compounds. As the monomer other than the (meth)acrylic acid ester, only one kind may be used, or two or more kinds thereof may be used in combination.

Examples of the (meth)acrylamides include (meth)acrylamide, N-isopropyl (meth)acrylamide, N-tert-butyl (meth)acrylamide, N,N'-dimethyl (meth)acrylamide, (3-(meth)acrylamide propyl)trimethylammonium chloride, 4-(meth)acryloylmorpholine, 3-(meth)acryloyl-2-oxazolidinone, N-[3-(dimethylamino)propyl](meth)acrylamide, N-(2-hydroxyethyl)(meth)acrylamide, N-methylol(meth)acrylamide, and 6-(meth)acrylamide hexanoic acid.

Examples of the vinyl compound include ethylene, allylamine, vinylpyrrolidone, maleic anhydride, maleimide, itaconic acid, (meth)acrylic acid, and vinylamine.

<Peptide moiety>

The peptide moiety is a structural portion derived from a peptide. The peptide moiety has an amino acid sequence. The peptide constituting the peptide moiety may be an oligopeptide or a polypeptide. As the peptide, only one kind may be used, or two or more kinds thereof may be used in combination.

The number of amino acid residues of the peptide moiety is preferably 3 or more, more preferably 4 or more, and still more preferably 5 or more, and preferably 10 or less, more preferably 8 or less, and still more preferably 6 or less. When the number of the amino acid residues is the above lower limit or more and the above upper limit or less, the adhesiveness to the seeded cells can be further enhanced, and the proliferation rate of the cells can be further enhanced. However, the number of amino acid residues of the peptide moiety may be more than 10 or more than 15.

The peptide moiety preferably has a cell adhesive amino acid sequence. The cell adhesive amino acid sequence refers to an amino acid sequence whose cell adhesion activity has been confirmed by a phage display method, a sepharose bead method, or a plate coating method. As the phage display method, for example, a method described in "The Journal of Cell Biology, Volume 130, Number 5, September 1995 1189-1196" can be used. As the sepharose bead method, for example, a method described in "Protein Nucleic Acid and Enzyme, Vol. 45 No. 15 (2000) 2477" can be used. As the plate coating method, for example, a method described in "Protein, Nucleic Acid and Enzyme, Vol. 45 No. 15 (2000) 2477" can be used.

Examples of the cell adhesive amino acid sequence include RGD sequence (Arg-Gly-Asp), YIGSR sequence (Tyr-Ile-Gly-Ser-Arg), PDSGR sequence (Pro-Asp-Ser-Gly-Arg), HAV sequence (His-Ala-Val), ADT sequence (Ala-Asp-Thr), QAV sequence (Gln-Ala-Val), LDV sequence (Leu-Asp-Val), IDS sequence (Ile-Asp-Ser), REDV sequence (Arg-Glu-Asp-Val), IDAPS sequence (Ile-Asp-Ala-Pro-Ser), KQAGDV sequence (Lys-Gln-Ala-Gly-Asp-Val), and TDE sequence (Thr-Asp-Glu). Examples of the cell adhesive amino acid sequence include sequences described in "Medicina Philosophica, Vol. 9, No. 7, pp. 527-535, 1990" and "Journal of Osaka Women's and Children's Hospital, Vol. 8, No. 1, pp. 58-66, 1992". The peptide moiety may have only one kind of the cell adhesive amino acid sequence, or may have two or more kinds thereof.

The cell adhesive amino acid sequence preferably has at least one of the above-described cell adhesive amino acid sequences, more preferably has at least an RGD sequence, a YIGSR sequence, or a PDSGR sequence, and still more preferably has at least an RGD sequence represented by the following formula (1). In this case, the adhesiveness to the seeded cells can be further enhanced, and the proliferation rate of the cells can be further enhanced.

Arg-Gly-Asp-X ... Formula (1)

In the above formula (1), X represents Gly, Ala, Val, Ser, Thr, Phe, Met, Pro, or Asn.

The peptide moiety may be linear or may have a cyclic peptide skeleton. From the viewpoint of further enhancing a cell-proliferating property, the peptide moiety preferably has a cyclic peptide skeleton. The cyclic peptide skeleton is a cyclic skeleton composed of a plurality of amino acids. From the viewpoint of more effectively exhibiting the effect of

the present invention, the cyclic peptide skeleton is preferably composed of 4 or more amino acids, and more preferably composed of 5 or more amino acids, and preferably composed of 10 or less amino acids.

In the peptide-conjugated resin, the content rate of the peptide moiety is preferably 0.1 mol% or more, more preferably 1 mol% or more, still more preferably 5 mol% or more, and particularly preferably 10 mol% or more, and preferably 60 mol% or less, more preferably 50 mol% or less, still more preferably 35 mol% or less, and particularly preferably 25 mol% or less. When the content rate of the peptide moiety is the above lower limit or more, the adhesiveness to the seeded cells can be further enhanced, and the proliferation rate of the cells can be further enhanced. When the content rate of the peptide moiety is the above upper limit or less, the production cost can be suppressed. The content rate (mol%) of the peptide moiety is the substance amount of the peptide moiety with respect to the sum of the substance amounts of respective structural units constituting the peptide-conjugated resin.

The content rate of the peptide moiety can be measured by, for example, NMR, FT-IR, or LC-MS.

<Linker moiety>

The linker moiety is a structural portion derived from a linker. The linker moiety is usually located between the polyvinyl alcohol derivative skeleton or the poly(meth)acrylic acid ester skeleton and the peptide moiety. The polyvinyl alcohol derivative skeleton or the poly(meth)acrylic acid ester skeleton and the peptide moiety are bonded via the linker moiety. The linker moiety is formed by a linker (crosslinking agent). As the linker, only one kind may be used, or two or more kinds thereof may be used in combination.

The linker is preferably a compound having a functional group capable of being bonded to the peptide, and more preferably a compound having a functional group capable of condensing with a carboxyl group or an amino group of the peptide.

Examples of the functional group capable of condensing with a carboxyl group or an amino group of the peptide include a carboxyl group, a thiol group, an amino group, a hydroxyl group, and a cyano group.

From the viewpoint of satisfactorily reacting with the peptide, the linker is preferably a compound having a carboxyl group or an amino group, and more preferably a compound having a carboxyl group.

In the case of obtaining the peptide-conjugated resin having a polyvinyl alcohol derivative skeleton, examples of the linker having a carboxyl group include (meth)acrylic acid and carboxyl group-containing acrylamide. By using a carboxylic acid having a polymerizable unsaturated group (carboxylic acid monomer) as the linker having a carboxyl group, the carboxylic acid monomer can be polymerized by graft polymerization at the time of introduction of the linker, so that the number of the carboxyl groups that can react with the peptide can be increased.

From the viewpoint of satisfactorily bonding the polyvinyl alcohol derivative and the peptide, the linker is preferably (meth)acrylic acid, and more preferably acrylic acid.

In the case of obtaining the peptide-conjugated resin having a poly(meth)acrylic acid ester skeleton, the linker preferably has a functional group capable of being bonded to a (meth)acrylic acid ester. Examples of the functional group capable of being bonded to the (meth)acrylic acid ester include a vinyl group, a (meth)acryloyl group, and an allyl group. The linker more preferably has a (meth)acryloyl group as the functional group capable of being bonded to the (meth)acrylic acid ester, and is preferably a compound having a carboxyl group or an amino group and having a (meth)acryloyl group.

Examples of the linker in the case of obtaining the peptide-conjugated resin having a poly(meth)acrylic acid ester skeleton include (meth)acrylic acid, itaconic acid, and acrylamide.

From the viewpoint of satisfactorily bonding the poly(meth)acrylic acid ester and the peptide, the linker is preferably (meth)acrylic acid or itaconic acid, and more preferably (meth)acrylic acid.

<Other details of coating layer>

The weight average molecular weight of the synthetic resin is preferably 10,000 or more, and more preferably 50,000 or more, and preferably 1,200,000 or less, and more preferably 600,000 or less. When the weight average molecular weight is the above lower limit or more and the above upper limit or less, the effect of the present invention can be more effectively exhibited. When the weight average molecular weight is the above upper limit or less, the extensibility of cells in cell culture can be more effectively enhanced.

The weight average molecular weight can be measured by, for example, the following method. The synthetic resin is dissolved in tetrahydrofuran (THF) to prepare a 0.2% by weight solution of the synthetic resin. Next, evaluation is performed using a gel permeation chromatography (GPC) measuring device (APC system, manufactured by Waters Corporation) under the following measurement conditions.

Column: HSPgel HR MB-M 6.0 × 150 mm

Flow rate: 0.5 mL/min
Column temperature: 40°C
Injection volume: 10 μL
Detector: RI, PDA
Standard sample: Polystyrene

The coating layer may contain only the resin having a polyvinyl alcohol derivative skeleton or a poly(meth)acrylic acid ester skeleton. The coating layer may contain the resin having a polyvinyl alcohol derivative skeleton or a poly(meth)acrylic acid ester skeleton and not having a peptide moiety, and the peptide-conjugated resin. The coating layer may contain other component such as a resin having neither the polyvinyl alcohol derivative skeleton nor the poly(meth)acrylic acid ester skeleton. Examples of the other component include a polyolefin resin, a polyether resin, polyester, an epoxy resin, a polyamide resin, a polyimide resin, a polyurethane resin, a polycarbonate resin, cellulose, and a polypeptide. As the other component, only one kind may be used, or two or more kinds thereof may be used in combination.

The coating layer may have only a layer containing the resin having a polyvinyl alcohol derivative skeleton or a poly(meth)acrylic acid ester skeleton (hereinafter, may be referred to as a "layer X"). The coating layer may have a layer not containing the resin having a polyvinyl alcohol derivative skeleton or a poly(meth)acrylic acid ester skeleton (hereinafter, may be referred to as a "layer Y") . The coating layer may have the layer X and the layer Y. When the coating layer has the layer X and the layer Y, it is preferable that the layer Y is located on the base particle side and the layer X is located outside the layer Y in the coating layer. In this case, the adhesiveness between the microcarrier and the cell can be further enhanced.

The peptide-conjugated resin is preferably present at least on the outer surface of the microcarrier. The outermost layer of the microcarrier is preferably a layer containing the peptide-conjugated resin. In this case, the adhesiveness between the microcarrier and the cell can be further enhanced.

The content of the synthetic resin in 100% by weight of the coating layer is preferably 90% by weight or more, more preferably 95% by weight or more, still more preferably 97.5% by weight or more, particularly preferably 99% by weight or more, and most preferably 100% by weight (whole amount). When the content of the synthetic resin is the above lower limit or more, the effect of the present invention can be more effectively exhibited. The content of the synthetic resin in 100% by weight of the coating layer may be 100% by weight or less, or may be less than 100% by weight.

The content of the resin having a polyvinyl alcohol derivative skeleton or a poly(meth)acrylic acid ester skeleton or the peptide-conjugated resin in 100% by weight of the coating layer is preferably 90% by weight or more, more preferably 95% by weight or more, still more preferably 97.5% by weight or more, particularly preferably 99% by weight or more, and most preferably 100% by weight (whole amount). When the content of the resin having a polyvinyl alcohol derivative skeleton or a poly(meth)acrylic acid ester skeleton or the peptide-conjugated resin is the above lower limit or more, the effect of the present invention can be more effectively exhibited. The content of the resin having a polyvinyl alcohol derivative skeleton or a poly (meth) acrylic acid ester skeleton or the peptide-conjugated resin in 100% by weight of the coating layer may be 100% by weight or less, or may be less than 100% by weight.

The surface area (coverage) covered with the coating layer in 100% of the total surface area of the base particle is preferably 50% or more, more preferably 70% or more, still more preferably 90% or more, yet still more preferably 95% or more, particularly preferably 99% or more, and most preferably 100%. When the coverage is the lower limit or more, the adhesiveness between the microcarrier and the cell can be further enhanced, and the effect of the present invention can be more effectively exhibited. The coverage may be 100% or less, less than 100%, or 99% or less.

The coverage is determined by observing the microcarrier with an electron microscope or an optical microscope and calculating the percentage of the surface area covered with the coating layer with respect to the projected area of the base particle.

The thickness of the coating layer is preferably 10 nm or more, and more preferably 50 nm or more, and preferably 1000 nm or less, and more preferably 500 nm or less. When the thickness of the coating layer is the above lower limit or more and the above upper limit or less, the adhesiveness between the microcarrier and the cell can be further enhanced. When the thickness of the coating layer is the above lower limit or more and the above upper limit or less, the effect of the present invention can be more effectively exhibited.

The thickness of the coating layer can be measured by observing the cross section of microcarriers using, for example, a scanning electron microscope (SEM). With regard to the thickness of the coating layer, it is preferable to calculate the average value of the thicknesses of optional five portions of the coating layer as the thickness of the coating layer of one microcarrier, and it is more preferable to calculate the average value of the thicknesses of the entire coating layer as the thickness of the coating layer of one microcarrier. The thickness of the coating layer is preferably determined by calculating the average value of the thicknesses of the coating layers of optional 50 microcarriers.

Examples of a method for obtaining the peptide-conjugated resin having a polyvinyl alcohol derivative skeleton include the following method.

A polyvinyl alcohol derivative (for example, a polyvinyl acetal resin) is reacted with a linker to obtain a reaction product in which the polyvinyl acetal resin and the linker are bonded to each other. The obtained reaction product is reacted with a peptide to obtain the peptide-conjugated resin having a polyvinyl alcohol derivative skeleton (polyvinyl acetal skeleton).

Examples of a method for obtaining the peptide-conjugated resin having a poly(meth)acrylic acid ester skeleton include the following method.

An acrylic resin obtained by polymerizing a monomer containing a (meth)acrylic acid ester is obtained. The obtained acrylic resin, a peptide, and a linker used as necessary are reacted to obtain the peptide-conjugated resin having a poly(meth)acrylic acid ester skeleton.

Examples of a method for obtaining the peptide-conjugated resin having a polyvinyl alcohol derivative skeleton and a poly(meth)acrylic acid ester skeleton include the following method.

A resin having a polyvinyl alcohol derivative skeleton and a poly(meth)acrylic acid ester skeleton is obtained by the following method (i), (ii) or (iii). (i) A polyvinyl acetal resin is synthesized using polyvinyl alcohol copolymerized with an acrylic acid ester. (ii) A polyvinyl acetal resin is synthesized using polyvinyl alcohol and polyvinyl alcohol copolymerized with an acrylic acid ester. (iii) An acrylic acid ester is graft-copolymerized with the polyvinyl acetal resin. The resin obtained by the method (i), (ii), or (iii), a peptide, and a linker used as necessary are reacted to obtain a peptide-conjugated resin having a polyvinyl alcohol derivative skeleton and a poly(meth)acrylic acid ester skeleton.

Examples of a method for obtaining a microcarrier by disposing the coating layer on the surface of the base particle include the following methods (1) and (2).

Method (1): The peptide-conjugated resin obtained by the above-described method is dissolved in a solvent to obtain a solution containing the peptide-conjugated resin. By spraying the solution containing the peptide-conjugated resin onto the base particle or separating the base particle impregnated with the solution containing the peptide-conjugated resin, a microcarrier including a layer (coating layer) containing the peptide-conjugated resin on the outer surface of the base particle can be produced.

Method (2): A resin having a polyvinyl alcohol derivative skeleton or a poly(meth)acrylic acid ester skeleton (resin before peptide bonding) is prepared. This resin is dissolved in a solvent to obtain a resin-containing solution. The resin-containing solution is sprayed onto the base particle or the base particle impregnated with the resin-containing solution is separated to obtain a particle in which a layer containing a resin having a polyvinyl alcohol derivative or a poly(meth)acrylic acid ester is disposed on the outer surface of the base particle. The obtained particle is reacted with the resin having a polyvinyl alcohol derivative or a poly(meth)acrylic acid ester contained in the layer, a peptide, and a linker used as necessary by the above-described method. Thus, a microcarrier can be produced, which includes a layer containing a resin having a polyvinyl alcohol derivative skeleton or a poly(meth)acrylic acid ester skeleton and not having a peptide moiety and a layer containing a peptide-conjugated resin as coating layers on the outer surface of the base particle.

(Other details of microcarrier)

The microcarrier is used for culturing cells.

Examples of the cells include cells of animals such as human, mouse, rat, pig, cow, and monkey. Examples of the cells include somatic cells such as stem cells, progenitor cells, and mature cells. The somatic cells may be cancer cells.

Examples of the stem cells include mesenchymal stem cells (MSCs), iPS cells, ES cells, Muse cells, embryonic cancer cells, embryonic germ stem cells, and mGS cells.

Examples of the mature cells include nerve cells, cardiomyocytes, retinal cells, and hepatocytes.

The microcarrier is preferably used for three-dimensional culture of cells. The three-dimensional culture is a culture method in which cells are cultured with a thickness also in a longitudinal direction with respect to two-dimensional culture in which cells are cultured on a plane such as a plate.

The microcarrier is preferably used for serum-free medium culture. In the microcarrier, the adhesiveness of cells can be enhanced even in a serum-free medium culture containing no feeder cell or adhesive protein, and in particular, the initial fixing rate after cell seeding can be further enhanced. In the microcarrier, the effect of the present invention can be exhibited even in the serum-free medium culture. In particular, when the coating layer contains the peptide-conjugated resin, an effect of enhancing the adhesiveness of cells even in the serum-free medium culture, and an effect of enhancing the initial fixing rate after cell seeding are effectively exhibited.

It is preferable that the microcarrier does not substantially contain an animal-derived raw material. Since it does not contain the animal-derived raw material, it is possible to provide a microcarrier having high safety and little variation in quality during production. The phrase "does not substantially contain an animal-derived raw material" means that the amount of the animal-derived raw material in the microcarrier is 3% by weight or less. In the microcarrier, the amount of the animal-derived raw material in the microcarrier is preferably 1% by weight or less, and most preferably 0% by

weight. That is, it is most preferable that the microcarrier does not contain any animal-derived raw material.

(Cell culturing method)

Cells can be cultured using the microcarrier. The cell culturing method according to the present invention is a cell culturing method using the microcarrier described above. Examples of the cells include the cells described above.
The cell culturing method preferably includes the step of causing cells to adhere to the microcarrier. The cells may be cell clumps. The cell clumps can be obtained by adding a cell-releasing agent to a confluent culture vessel and uniformly crushing them by pipetting. The cell-releasing agent is not particularly limited, but an ethylenediamine/phosphate buffer solution is preferable. The size of the cell clumps is preferably 50 um to 200 μm.
The cell culturing method preferably includes the step of shaking or stirring the microcarriers to which the cells adhere in a culture medium. The step of shaking or stirring the microcarriers in the culture medium is preferably the step of suspending the microcarriers to which the cells adhere in the culture medium. In the step of suspending the microcarriers to which the cells adhere in the culture medium, it is preferable to suspend all the microcarriers, but some of the microcarriers may remain at the bottom of the vessel.
The cell culturing method preferably includes the steps of settling the microcarriers to which the cells adhere and replacing a culture medium. The step of settling the microcarriers is preferably the step of settling the microcarriers to which the cells adhere by gravity. In the step of settling the microcarriers, all the microcarriers may be settled to the bottom of the culture vessel, or some of the microcarriers may be in a state of being suspended in the culture medium. The step of replacing the culture medium preferably includes the steps of removing a supernatant culture medium and adding a new culture medium. In the step of removing the supernatant culture medium, the culture medium positioned above the settled microcarriers is removed. From the viewpoint of suppressing the removal of the microcarriers to which the cells adhere and from the viewpoint of enhancing the efficiency of culturing the cells, it is preferable that the number of the microcarriers remaining suspended in the culture medium is smaller after the step of settling the microcarriers is completed and before the step of removing the supernatant culture medium is started.
Hereinafter, the present invention will be described in more detail with reference to Examples and Comparative Examples. The present invention is not limited only to these Examples.
The content rate of structural units in the obtained resin was measured by $^1$H-NMR (nuclear magnetic resonance spectrum) after a synthetic resin was dissolved in DMSO-d6 (dimethyl sulfoxide).

(Example 1)

(1) Production of base particle A

Eight hundred parts by weight of divinylbenzene (purity: 57%) and 200 parts by weight of styrene were mixed to obtain a mixed solution. To the obtained mixed solution, 20 parts by weight of benzoyl peroxide was added, and the resulting mixture was stirred until it was uniformly dissolved to obtain a monomer mixed solution. In a reaction kettle, 4000 parts by weight of a 2% by weight aqueous solution obtained by dissolving polyvinyl alcohol having a molecular weight of about 1700 in pure water was put. Then, the obtained monomer mixed solution was put in the reaction kettle and stirred for 4 hours to adjust the particle size of monomer droplets so that the monomer droplets had a predetermined particle size. Then, the reaction was performed in a nitrogen atmosphere at 85°C for 9 hours to perform the polymerization reaction of the monomer droplets, thereby obtaining a particle. The obtained particle was washed several times with hot water, methanol, and acetone, respectively, and then classified to obtain a base particle A having an average particle diameter of 100 um and a particle diameter CV value of 1%. The base particle A is a resin particle made of a divinylbenzene copolymer (described as DVB in Table).

(2) Production of polyvinyl acetal resin

Into a reactor equipped with a stirrer, 2700 mL of ionexchanged water, 300 parts by weight of polyvinyl alcohol having an average polymerization degree of 1700 and a saponification degree of 99 mol% were charged, and dissolved by heating with stirring to obtain a solution. To the obtained solution, 35% by weight hydrochloric acid was added as a catalyst so that the concentration of hydrochloric acid became 0.2% by weight. Then, the temperature was adjusted to 15°C, and 22 parts by weight of n-butyraldehyde was added thereto with stirring. Then, 148 parts by weight of n-butyraldehyde was added thereto to precipitate a white particulate polyvinyl acetal resin (polyvinyl butyral resin). After 15 minutes from the precipitation, 35% by weight hydrochloric acid was added so that the concentration of hydrochloric acid became 1.8% by weight, and the mixture was then heated to 50°C and held at 50°C for 2 hours. Then, the solution was cooled and neutralized, and the polyvinyl butyral resin was then washed with water and dried to obtain a polyvinyl acetal resin (polyvinyl butyral resin, average polymerization degree: 1700, acetalization degree (butyralization degree):

70 mol%, amount of hydroxyl groups: 27 mol%, acetylation degree: 3 mol%).

(3) Formation of linker moiety

Nighty nine parts by weight of the obtained polyvinyl acetal resin and 1 part by weight of acrylic acid (linker) were dissolved in 300 parts by weight of THF (tetrahydrofuran), and reacted under ultraviolet irradiation for 20 minutes in the presence of a photoradical polymerization initiator to graftcopolymerize the polyvinyl acetal resin and the acrylic acid, thereby forming a linker moiety.

(4) Production of polyvinyl acetal resin-coated particle having linker moiety formed

One part by weight of the polyvinyl acetal resin having the linker moiety formed was dissolved in 19 parts by weight of butanol. One part by weight of the base particle A was added to the obtained solution. The mixture was stirred, then filtered, washed with pure water, and vacuum-dried at 60°C for 5 hours to obtain a polyvinyl acetal resin-coated particle having the linker moiety formed.

(5) Production of microcarrier

A linear peptide having an amino acid sequence of Gly-Arg-Gly-Asp-Ser (the number of amino acid residues: 5) was prepared. One part by weight of this peptide and 1 part by weight of 1-ethyl-3-(3-dimethylaminopropyl)carbodiimide hydrochloride (condensing agent) were added to phosphate buffered saline containing neither calcium nor magnesium so that the final concentration of the peptide became 1 mM, thereby producing a peptide-containing solution. To 20 parts by weight of the obtained peptide-containing solution was added 1 part by weight of the polyvinyl acetal resin-coated particle having a linker moiety formed, so that a carboxyl group of the linker moiety and an amino group of Gly of the peptide were dehydrated and condensed. The obtained suspension was filtered, washed with pure water, and vacuum-dried at 60°C for 5 hours to obtain microcarriers. In Table, a peptide-conjugated resin having a polyvinyl alcohol derivative skeleton (polyvinyl acetal skeleton) obtained by the above-described method was described as a resin X1. The resin X1 has an amino acid sequence of Gly-Arg-Gly-Asp-Ser as a peptide moiety.

(Example 2)

(1) Production of base particle B

A polymerization reaction was performed in the same manner as in Example 1 to obtain a particle. The obtained particle was classified to obtain a base particle B having an average particle diameter of 200 um and a particle diameter CV value of 1%.

(2) Production of microcarrier

A microcarrier was produced in the same manner as in Example 1 except that the obtained base particle B was used.

(Example 3)

(1) Production of base particle C

A polymerization reaction was performed in the same manner as in Example 1 to obtain a particle. The obtained particle was classified to obtain a base particle C having an average particle diameter of 300 um and a particle diameter CV value of 1%.

(2) Production of microcarrier

A microcarrier was produced in the same manner as in Example 1 except that the obtained base particle C was used.

(Example 4)

(1) Production of base particle D

A polymerization reaction was performed in the same manner as in Example 1 to obtain a particle. The obtained particle was classified to obtain a base particle D having an average particle diameter of 400 um and a particle diameter CV value of 1%.

(2) Production of microcarrier

A microcarrier was produced in the same manner as in Example 1 except that the obtained base particle D was used.

(Example 5)

(1) Production of base particle E

Micropearl GS-L300 (polyfunctional acrylic resin particle manufactured by Sekisui Chemical Co., Ltd., average particle diameter: 300 um, particle diameter CV value: 7%) was prepared. This particle was classified to obtain a base particle E having an average particle diameter of 300 um and a particle diameter CV value of 1%. The base particle E is a resin particle made of an acrylic resin (described as ACR in Table).

(2) Production of microcarrier

A microcarrier was produced in the same manner as in Example 1 except that the obtained base particle E was used.

(Example 6)

(1) Production of base particle F

Carbon black having a surface coated with a polymer was prepared. Five parts by weight of this carbon black, 47.5 parts by weight of divinylbenzene, and 47.5 parts by weight of tetramethylolmethane triacrylate were mixed to obtain a dispersion. To this dispersion, 20 parts by weight of lauroyl peroxide was added, followed by uniformly mixing to obtain a mixed solution. The obtained mixed solution was added to 8500 parts by weight of a 3% by weight aqueous polyvinyl alcohol solution, followed by sufficiently stirring. The stirred product was then adjusted to have a predetermined emulsified diameter with a homogenizer to obtain an emulsion. The emulsion was transferred to a 20 liter reaction kettle equipped with a thermometer, a stirrer, and a reflux condenser, and heated to 85°C while being stirred in a nitrogen atmosphere to perform a polymerization reaction for 7 hours. Next, the polymerization reaction solution was cooled, and the obtained particle was washed with hot water, methanol, and acetone several times. The obtained particle was classified to obtain a base particle F having an average particle diameter of 300 um and a particle diameter CV value of 1%. The base particle F is a resin particle made of a divinylbenzene copolymer (containing carbon black as an inorganic filler, described as +CB in Table).

(2) Production of microcarrier

A microcarrier was produced in the same manner as in Example 1 except that the obtained base particle F was used.

(Example 7)

The base particle C was used as the base particle.

(2) Production of acrylic resin

Ten parts by weight of dodecyl acrylate and 2.7 parts by weight of acrylic acid were dissolved in 27 parts by weight of tetrahydrofuran to obtain an acrylic monomer solution. In the obtained acrylic monomer solution, 0.0575 parts by weight of Irgacure 184 (manufactured by BASF SE) was dissolved, and the obtained solution was applied onto a

PET film. The applied product was irradiated with light having a wavelength of 365 nm at an integrated light amount of 2000 mJ/cm$^2$ using a UV conveyor device ("ECS301G1" manufactured by Eye Graphics Co., Ltd.) at 25°C to obtain a (meth)acrylic copolymer solution. The obtained (meth)acrylic copolymer solution was vacuum-dried at 80°C for 3 hours to obtain an acrylic resin having a linker moiety.

(3) Production of acrylic resin-coated particle

One part by weight of the obtained acrylic resin was dissolved in 19 parts by weight of butanol. One part by weight of the base particle was added to this solution, followed by stirring. The stirred product was then filtered, washed with pure water, and vacuum-dried at 60°C for 5 hours to obtain an acrylic resin-coated particle.

(4) Production of microcarrier

The obtained acrylic resin-coated particle was used. A cyclic peptide having an amino acid sequence of Arg-Gly-Asp-Phe-Lys (the number of amino acid residues: 5, cyclic skeleton formed by bonding Arg and Lys, D-form Phe) was prepared as a peptide. A microcarrier was obtained in the same manner as in Example 1 except that a carboxyl group in a structural unit derived from acrylic acid of the acrylic resin and an amino group of Lys of the peptide were dehydrated and condensed using this peptide. In Table, a peptide-conjugated resin having a poly(meth)acrylic acid ester skeleton obtained by the above-described method was described as a resin X2. The resin X2 has an amino acid sequence of Arg-Gly-Asp-Phe-Lys (cyclic peptide skeleton) as a peptide moiety.

(Comparative Example 1)

(1) Production of base particle G

A particle obtained by classifying an "untreated microcarrier" (base particle: polystyrene particle, described as PS in Table) manufactured by Corning Incorporated and having a particle diameter CV value set to 1% was used as a base particle G.

(2) Production of microcarrier

A microcarrier was produced in the same manner as in Example 1 except that the base particle G was used.

(Comparative Example 2)

The obtained base particle C itself was used as a microcarrier.

(Reference Example A)

A particle obtained by classifying "Low-Concentration Synthemax II Microcarrier " (a microcarrier in which a base particle was a PS particle and a coating layer was fibronectin) manufactured by Corning Incorporated and having a particle diameter CV value set to 1% was used as it was.

(Evaluation)

(1) Specific Gravities of Base Particle and Microcarrier

The specific gravities of the obtained base particle and microcarrier were measured in a dry state and under an argon gas atmosphere using a true specific gravity meter ("Accupyc II" manufactured by Shimadzu Corporation).

(2) Average particle diameter of microcarrier and coefficient of variation in particle diameter (CV Value)

The obtained microcarrier was observed with a scanning electron microscope. The average particle diameter of the equivalent circle diameters of optional 50 microcarriers and the coefficient of variation (CV value) in the particle diameter were calculated.

(3) Thickness of coating layer

The cross section of the obtained microcarrier was observed with a scanning electron microscope. The thicknesses of the coating layers were measured for the optional 50 microcarriers, and the average value thereof was taken as the thickness of the coating layer of the microcarrier.

(4) Water absorption of microcarrier

The obtained microcarrier was dried in an oven at 100°C for 8 hours. One hundred point zero mg of the microcarrier was weighed, and allowed to stand for 24 hours in an environment of a temperature of 37°C and a relative humidity of 95% RH. The weight of the microcarrier after being allowed to stand was measured, and the water absorption of the microcarrier was calculated by the following formula.

$$\text{Water absorption (\% by weight)} = (W_2 - W_1)/W_1 \times 100$$

$W_1$: Weight of microcarrier before standing (mg)
$W_2$: Weight of microcarrier after standing (mg)

(5) Settling time of microcarrier

A PBS buffer solution was put in a 100 mL graduated cylinder to a depth of 60 cm. Five hundred mg of the obtained microcarrier was gently charged from a height of 20 cm from a water surface. A time from the charge until the microcarrier was completely settled to the bottom of the graduated cylinder was measured.

(6) Cell culture

A T flask (manufactured by Corning Incorporated, volume: 60 mL, no surface treatment) was used as a culture vessel. The following liquid culture medium and ROCK (Rho-linked kinase) specific inhibitor were prepared.

STEMFIT culture medium (manufactured by Ajinomoto Healthy Supply Inc.)
ROCK-Inhibitor (Y27632)

Into a $\phi$35 mm dish, $3.8 \times 10^4$ h-iPS cells 201B7 in a confluent state, and 1 mL of a 0.5 mM ethylenediaminetetraacetic acid/phosphate buffer solution were added, and the mixture was allowed to stand at room temperature for 5 minutes. The ethylenediaminetetraacetic acid/phosphate buffer solution was removed, followed by pipetting with 1 mL of a liquid culture medium to obtain a cell suspension. The whole amount of the obtained cell suspension was added into a culture vessel containing the obtained microcarrier (150 $cm^2$ in terms of surface area) and 30 mL of a liquid culture medium. The culture vessel was set in a shaker, and shaken for culture at 37°C, a $CO_2$ concentration of 5%, and 46 rpm.

(6-1) Aggregation of microcarrier due to cell clumps

After three days from the start of the shaking culture, the culture vessel was taken out, and allowed to stand until the microcarriers were completely settled. A supernatant liquid culture medium was then replaced with a new liquid culture medium. The microcarrier after the culture medium replacement was collected, and optional 50 microcarriers were observed with a phase contrast microscope. The number of microcarriers present independently without adhering to adjacent microcarriers via cell clumps was counted.

<Determination criteria of aggregation of microcarrier due to cell clumps>

AA: The number of microcarriers present independently is 45 or more.
A: The number of microcarriers present independently is 40 or more and 44 or less.
B: The number of microcarriers present independently is 30 or more and 39 or less.
C: The number of microcarriers present independently is 29 or less.

(6-2) Evaluation of cell culture (number of cells)

After five days from the start of the shaking culture, 30 ml of a supernatant liquid culture medium was removed from the culture vessel. Then, 7.5 mL of a TryPLE Express releasing solution was added to the culture vessel, followed by suspending by performing a pipetting operation. The suspension was added to a cell strainer together with the microcarriers to separate the microcarriers from the cell suspension. The number of cells contained in the obtained cell suspension was determined using a cell counter ("NucleoCounter NC-3000" manufactured by Chemometec).

<Determination criteria of evaluation of cell culture>

AA: The number of cells is $4.0 \times 10^5$ or more.
A: The number of cells is $3.0 \times 10^5$ or more and less than $4.0 \times 10^5$.
B: The number of cells is $1.0 \times 10^5$ or more and less than $3.0 \times 10^5$.
C: The number of cells is less than $1.0 \times 10^5$.

The details and results are shown in Tables 1 and 2 below.

[Table 1]

| | | | Example 1 | Example 2 | Example 3 | Example 4 | Example 5 |
|---|---|---|---|---|---|---|---|
| Base particle | Kind of base particle | | A | B | C | D | E |
| | Kind of resin | | DVB | DVB | DVB | DVB | ACR |
| | Average particle diameter | μm | 100 | 200 | 300 | 400 | 300 |
| | Specific gravity | $g/cm^3$ | 1.11 | 1.11 | 1.11 | 1.11 | 1.19 |
| Coating layer | Kind of resin | | Resin X1 | Resin X1 | Resin X1 | Resin X1 | Resin X1 |
| | Thickness | nm | 100 | 100 | 100 | 100 | 100 |
| Microcarrier | Specific gravity | $g/cm^3$ | 1.11 | 1.11 | 1.11 | 1.11 | 1.19 |
| | Average particle diameter | um | 100 | 200 | 300 | 400 | 300 |
| | Particle diameter CV value | % | 1 | 1 | 1 | 1 | 1 |
| | Water absorption | a by weight | 0.5 | 0.5 | 0.5 | 0.5 | 0.5 |
| Settling time of microcarrier | | | 1.5 minutes | 50 seconds | 15 seconds | 10 seconds | 10 seconds |
| Aggregation of microcarrier due to cell clumps | | | A | A | AA | AA | AA |
| Evaluation of cell culture (number of cells) | | | A | A | AA | AA | AA |

[Table 2]

| | | | Example 6 | Example 7 | Comparative Example 1 | Comparative Example 2 | Reference Example A |
|---|---|---|---|---|---|---|---|
| Base particle | Kind of base particle | | F | C | G | C | - |
| | Kind of resin | | +CB | DVB | PS | DVB | PS |
| | Average particle diameter | um | 300 | 300 | 200 | 300 | 200 |
| | Specific gravity | g/cm$^3$ | 1.29 | 1.11 | 1.02 | 1.11 | 1. 02 |
| Coating layer | Kind of resin | | Resin X1 | Resin X2 | Resin X1 | - | Protein |
| | Thickness | nm | 100 | 100 | 100 | - | 100 |
| Microcarrier | Specific gravity | g/cm$^3$ | 1.29 | 1.11 | 1.02 | 1.11 | 1. 02 |
| | Average particle diameter | μm | 300 | 300 | 200 | 300 | 200 |
| | Particle diameter CV value | % | 1 | 1 | 1 | 1 | 1 |
| | Water absorption | a by weight | 0.5 | 0.5 | 0.5 | 0.5 | - |
| Settling time of microcarrier | | | 5 seconds | 15 seconds | > 3 minutes | 15 seconds | > 3 minutes |
| Aggregation of microcarrier due to cell clumps | | | AA | AA | B | AA | B |
| Evaluation of cell culture (number of cells) | | | AA | AA | A | C | A |

**EXPLANATION OF SYMBOLS**

1: Cell culture microcarrier
2: Base particle
3: Coating layer

**Claims**

**1.** A cell culture microcarrier comprising:

a base particle; and
a coating layer coating an outer surface of the base particle,
the cell culture microcarrier having a specific gravity of 1.10 g/cm$^3$ or more.

**2.** The cell culture microcarrier according to claim 1, wherein the cell culture microcarrier has a water absorption of 10% by weight or less.

**3.** The cell culture microcarrier according to claim 1 or 2, wherein the coating layer contains a synthetic resin.

4. The cell culture microcarrier according to claim 3, wherein the synthetic resin has a polyvinyl alcohol derivative skeleton or a poly(meth)acrylic acid ester skeleton.

5. The cell culture microcarrier according to any one of claims 1 to 4, wherein the coating layer contains a peptide moiety.

6. The cell culture microcarrier according to any one of claims 1 to 5, wherein the base particle is a resin particle.

7. The cell culture microcarrier according to any one of claims 1 to 6, wherein the base particle contains a polymer of a monomer having an ethylenically unsaturated group.

8. The cell culture microcarrier according to claim 7, wherein the polymer of a monomer having an ethylenically unsaturated group is an acrylic resin, a divinylbenzene polymer, or a divinylbenzene copolymer.

9. The cell culture microcarrier according to any one of claims 1 to 8, wherein the cell culture microcarrier has an average particle diameter of 100 um or more and 1500 μm or less.

10. The cell culture microcarrier according to any one of claims 1 to 9, wherein a particle diameter CV value is 10% or less.

11. A cell culturing method comprising the steps of:

causing a cell to adhere to the cell culture microcarrier according to any one of claims 1 to 10;
settling the cell culture microcarrier to which the cell adheres; and
replacing a culture medium.

[FIG. 1.]

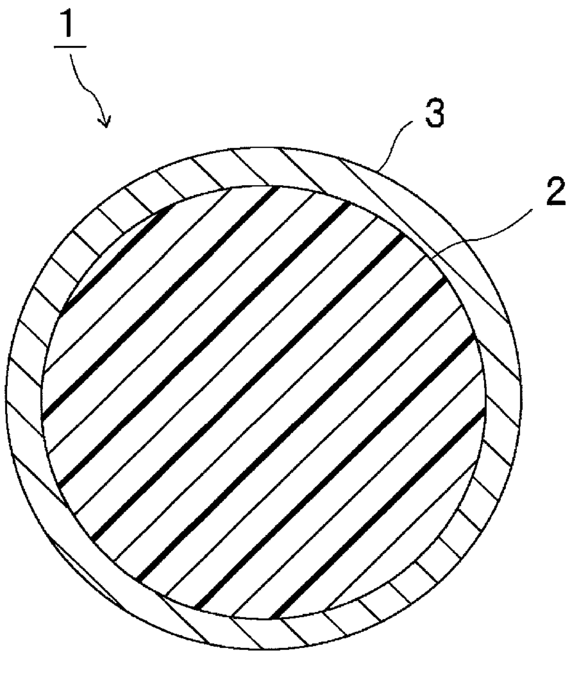

**INTERNATIONAL SEARCH REPORT**

International application No.
**PCT/JP2022/004062**

**A. CLASSIFICATION OF SUBJECT MATTER**

***C12N 5/02***(2006.01)i; ***C07K 5/00***(2006.01)i; ***C07K 7/06***(2006.01)i; ***C12M 3/00***(2006.01)i; ***C12N 5/071***(2010.01)i
FI: C12N5/02 ZNA; C07K5/00; C07K7/06; C12N5/071; C12M3/00 A

According to International Patent Classification (IPC) or to both national classification and IPC

**B. FIELDS SEARCHED**

Minimum documentation searched (classification system followed by classification symbols)

C12N5/02; C07K5/00; C07K7/06; C12M3/00; C12N5/071

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | JP 2011-030453 A (SANYO CHEM. IND., LTD.) 17 February 2011 (2011-02-17)<br>paragraphs [0006]-[0045], examples 1-4 | 1, 5, 6, 9, 11<br><br>2-11 |
| Y | JP 2016-171794 A (NATIONAL INST. OF ADVANCED INDUSTRIAL SCIENCE AND TECHNOLOGY) 29 September 2016 (2016-09-29)<br>paragraphs [0091], [0113]-[0132] | 2-11 |
| Y | JP 2013-500717 A (CORNING INC.) 10 January 2013 (2013-01-10)<br>paragraphs [0025]-[0028], [0068]-[0075], table 1 | 2-11 |
| A | 第2節 三次元培養時における方法の選択と低コスト化, iPS細胞の安全・高品質な作製技術, 第1版第1刷, 株式会社技術情亮協会, 31 October 2016, pp. 192-200, (TECHNICAL INFORMATION INSTITUTE CO., LTD.), non-official translation (Section 2 Selection of methods and cost reduction during 3D culture. Safe and High-Quality Production Technology for iPS Cells. first edition, first printing)<br>pp. 192, 193, table 1 | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 March 2022** | **29 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

INTERNATIONAL SEARCH REPORT

International application No.
PCT/JP2022/004062

C. DOCUMENTS CONSIDERED TO BE RELEVANT

| Category* | Citation of document, with indication, where appropriate, of the relevant passages | Relevant to claim No. |
|---|---|---|
| A | JP 6-038730 A (SAKAI ENETSUKUSU KK) 15 February 1994 (1994-02-15) entire text | 1-11 |

Form PCT/ISA/210 (second sheet) (January 2015)

INTERNATIONAL SEARCH REPORT

International application No.
**PCT/JP2022/004062**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2022/004062**

| Patent document cited in search report | Publication date (day/month/year) | Patent family member(s) | Publication date (day/month/year) |
|---|---|---|---|
| JP 2011-030453 A | 17 February 2011 | (Family: none) | |
| JP 2016-171794 A | 29 September 2016 | (Family: none) | |
| JP 2013-500717 A | 10 January 2013 | US 2011/0027889 A1<br>pp. 3-6, 8-10<br>WO 2011/017050 A1<br>EP 2459701 A1 | |
| JP 6-038730 A | 15 February 1994 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011017167 A1
- WO 2011017050 A1


### Non-patent literature cited in the description


- *The Journal of Cell Biology,* September 1995, vol. 130 (5), 1189-1196
- *Protein Nucleic Acid and Enzyme,* 2000, vol. 45 (15), 2477
- *Protein, Nucleic Acid and Enzyme,* 2000, vol. 45 (15), 2477
- *Medicina Philosophica,* 1990, vol. 9 (7), 527-535
- *Journal of Osaka Women's and Children's Hospital,* 1992, vol. 8 (1), 58-66